# EUROPEAN PATENT APPLICATION

(11) **EP 2 535 038 A2**
(43) Date of publication of application: **19.12.2012**
(21) Application number: 12165181.4
(22) Date of filing: 23.04.2012
(51) Int. Cl.: A61K 8/22, A61K 8/37, A61K 8/44, A61K 8/46, A61K 8/898, A61Q 5/06

(54) **Hair styling composition and method for styling hair**

(30) Priority: 21.04.2011 GB 201106789
(71) Applicant: PZ Cussons (International) Limited, Manchester M22 5TG (GB)
(72) Inventor: Cornwell, Paul, Chester, CH3 7QF (GB); Whatmough, Marie, Bacup, Lancashire, OL13 8HJ (GB); Green, Phillip, Stockport, SK3 0TX (GB)
(74) Representative: Wilson Gunn

(57) **Abstract**

The present invention relates to a system for a hair styling treatment which is able to semi-permanently straighten hair and control frizz. This treatment is able to straighten and smooth hair for up to about 4-6 weeks, significantly reduce hair styling times and improve the humidity resistance of straightened hair. The formulations described herein are able to achieve this with limited malodour.

## Description

The present invention relates to a system for a hair styling treatment which is able to semi-permanently straighten hair and control frizz. This treatment is able to straighten and smooth hair for up to about 4-6 weeks, significantly reduce hair styling times and improve the humidity resistance of straightened hair. The formulations described here are able to achieve this with limited malodour.

Managing, controlling and styling unruly hair is something faced by many people on a daily basis. A major concern to consumers is the significant amount of time which is regularly spent using blow-dryers and straightening irons in order to achieve the desired look. Another concern is frizzing and loss of style, experienced by consumers when their straightened hair is exposed to moisture.

Hair styling products have been commercially available for many years in the form of sprays, mousses, gels, balms and serums. However, these products are only able to provide a temporary solution to the problem of hair manageability and style hold, and their effects are removed when a person next washes their hair with shampoo. This means a person has to repeat the whole process after every wash.

In order to overcome these problems, it is common in the art for hair to be chemically treated and semi-permanently straightened. Typically, such reducing agent treatments involve breaking some of the disulphide linkages in the keratin in the hair, re-shaping the hair in some manner, and then subsequently recombining the disulphide linkages in a new configuration.

The most widely used reducing agent in permanent waving and straightening products is thioglycolic acid and its salts. However, thioglycolic acid and its salts can act as an irritant to a person's skin and can also have a strong unpleasant odour. A further disadvantage of systems based on thioglycolic acid is that oxidizing compositions, or neutralisers, are required for carrying out the fixation stage, and most such neutralisers contain high levels (5-10% w/w) of hydrogen peroxide. The use of high levels of hydrogen peroxide in the neutraliser is associated with deterioration of hair colour. The deterioration in hair colour makes it difficult for many women to use these products.

Whilst the use of reducing agents, such as thioglycolic acid and cysteamine, is very useful in straightening hair and controlling frizz, it is also important, in order to get the best results from a straightening regime, to include frizz control ingredients in a second, finishing product. Not all conditioning agents and silicones perform well to control frizz in this situation. Use of inefficient frizz-control ingredients can lead to consumer dissatisfaction.

It would therefore be desirable to provide system for a hair styling treatment which is suitable for personal home-use, that gives good hair straightening and frizz control that can last for up to about 4-6 weeks, but without one or more of the disadvantages associated with standard, thioglycolic acid-based, hair styling and straightening compositions and systems, such as the excessive malodour, skin irritation, and hair discolouration.

It has been found that these aims can be achieved through the use of cysteamine, or a cosmetically acceptable derivative thereof, as the primary reducing agent in a hair styling or straightening treatment.

Therefore, in accordance with the present invention, there is provided a system for styling hair, the system comprising the use of:
a) a composition comprising about 1 to about 10 wt% of cysteamine, or a cosmetically acceptable derivative thereof as a primary reducing agent; and
b) a composition comprising about 0.01% to about 2 wt.% of hydrogen peroxide, a cationic silicone compound, and a fatty acid ester.

Typically, the system of the invention is for semi-permanently straightening hair, and controlling frizz.

By 'cosmetically acceptable' it is meant herein any cysteamine derivative which substantially does not cause any adverse or undesirable reactions with a person's hair or skin when used in such a composition.

By 'primary reducing agent' it is meant herein that the composition (a) does not contain any other, or secondary, reducing agent in an amount which is greater than the amount of the cysteamine or a cosmetically acceptable derivative thereof, which is present in the composition.

The composition (a) in the system of the invention comprises cysteamine, or a cysteamine derivative, which is included as the primary reducing agent to cleave the disulphide linkages present in hair.

Typically, the primary reducing agent is cysteamine itself in its free form, or in the form of a cosmetically acceptable salt, such as cysteamine hydrochloride. Alternatively, cysteamine derivatives can be used. Non-limiting examples of cysteamine derivatives include monoalkyl aminoethanethiols (*e.g.* N-acyl cysteamine), dialkyl aminoethanethiols (*e.g.* 2-(dimethylamino)ethanethiol and 2-(diethylamino)ethanethiol) and others (e.g. tert-butyl N-(2-mercaptoethyl) carbamate). Alternatively, a combination of one or more of these may be used.

According to a further embodiment, the composition (a) in the system of the invention may contain one or more additional secondary reducing agents that are typically used in hair perm treatments. These secondary reducing agents are always present in an amount which is less that the amount of the cysteamine or derivative thereof which is in the composition (a). The secondary reducing agents are generally added at a level of about 10% or less, more typically at a level of about 9% or about 8% or less, by weight of the total composition (a). Non-limiting examples of these reducing agents include thiols such as thioglycolic acid, glyceryl monothioglycolate, cysteine, cysteamine, glutathione, and glyceryl thiolactate, or combinations of any two or more thereof.

Typically, the total amount of the cysteamine or a cysteamine derivative in composition (a) in the system of the invention may range from about 4% to about 7% by weight, based upon the total weight of the composition.

The use of cysteamine as a reducing agent in hair curling treatments is known. For example, Wella Patents EP261387A1, EP261387B1, WO1988001860A1 disclose the use of combinations of thioglycolate and cysteamine in treatments for curling hair.

However, it was not until the early 1990's that researchers started looking at the advantages of using cysteamine on its own as the sole reducing agent for hair curling treatments.

L'Oreal patent applications US5989534A, EP660700A1 and WO1995002391A1 disclose a two-step curling treatment that involves (1) applying a reducing agent treatment, containing just cysteamine, at a 5-20% active level, and (2) heating the hair on the rollers to 30-60°C to accelerate the reaction. The applications claim that no neutralising treatment is required, and that the cysteamine is simply rinsed away. They suggest that disulphide breakage by cysteamine is a fully reversible reaction, and that the absence of a neutralising step means less damage for the hair.

Helene Curtis patent applications US5601813A, US5460809A, US53824226A, US5260054A and EP1993100240A disclose a one-step curling treatment that involves treating the hair with 7.8-9.0% active cysteamine, with or without a heat step. Again, these patent applications suggests that the reactions with cysteamine are fully reversible and do not require a neutralising treatment. Test data shown in these applications further suggest that the cysteamine treatment does not significantly damage the hair.

Despite the advances made in L'Oreal and Helene Curtis, there were still problems with malodour associated with cysteamine based curling treatments. To try to tackle this L'Oreal patented a post-treatment formulation containing acids and fragrance masking ingredients to mask malodour (WO1994002114A1). Helene Curtis masked malodour with a polyhydric phenol (including resorcinol and derivatives, in US5554364A, WO1996040045A1).

Aside from the key patent applications mentioned already, there were also many others that mention the use of cysteamine. For example, Henkel disclosed the use of cysteamine in perm treatments in combination with mercaptoethanol (another reducing agent) and other ingredients (EP362663A1, WO1990003780A1). Showa Denko disclosed the use of cysteamine in perm treatments in combination with carbonyl compounds (e.g. 2-mercapto-4-butyrolactam) and other ingredients (WO2006068276A1; EP1827370A1). Dow Brands disclosed the use of cysteamine and other thio compounds (US5332570A). Wella disclosed a cysteamine derivative, N-mesyl cysteamine, as a reducing agent (EP723771A2, EP723771A3). Evans disclosed the use cysteamine and cysteine for curling and straightening (US5639451A), and the use of cysteamine, cysteine and a reducing sugar (EP1435901B1, EP1435901A1, WO2002092036A1). L'Oreal disclosed cysteamine and a stable polymer (EP432054B1), N-acyl cysteamine and a thioglycolate ester (US5225191A, EP440547A1), cysteamine and a carbonyl compound (US20080085251A1), N-acyl cysteamine and a thioglycolate ester (EP440547B1) and cysteamine derivatives (US5208014A, EP432051A1). Helene Curtis disclosed cysteamine and other reducing agents (US5362487A, US5439675A, US5456707A). Revlon patented a three-step system including a cysteamine treatment (12-18% active), a heating step and a neutraliser treatment (US5533532A).

It has however been surprisingly found that cysteamine, in its free form or as a derivative thereof, such as the hydrochloride salt, or in the form of N-acyl cysteamine, when used in combination with a 'neutralising conditioner' composition comprising hydrogen peroxide and the silicone and fatty acid ester as detailed hereinabove as composition (b), and a set of straightening irons, can deliver particularly good hair straightening effects at concentrations of 4.0-7.0% by weight of the total composition. Furthermore, these effects last for up to about 4-6 weeks.

Previous work with cysteamine hydrochloride, carried out by Nandagiri et al. on hair curling treatments, disclosed in US Patent Application 5,382,426, suggested that levels of 7.8-9.0% w/w are needed to provide good curl set. Work on cysteamine hydrochloride, carried out by Samain, also on curling treatments and disclosed in US Patent Application 5,989,534, suggested that levels of 5-20% w/w cysteamine hydrochloride are needed for good curl hold. The present inventors have however found that that a lower concentration of cysteamine or a derivative thereof is required to straighten hair, in comparison with the levels required to provide curl set.

The hydrogen peroxide in composition (b) forms part of a neutralising conditioner in the system of the invention, and acts as a primary odour neutralising ingredient.

According to a further embodiment, the composition (b) of the invention may contain one or more additional secondary odour neutralising ingredients. Non-limiting examples include fragrance ingredients, polyhydric phenols (including resorcinol), cyclodextrins, cationic polymeric materials (including cationised cellulose), zinc compounds (including zinc oxide, zinc rincinoleate, zinc carboxylate and zinc acetate), undecylenic acid, zeolites and silicas, or combinations of any two or more thereof. These secondary odour neutralising ingredients are always present in an amount which is less that the amount of the hydrogen peroxide which is in the composition (b).

Composition (b), however, contains hydrogen peroxide as the primary odour neutralising ingredient. The total amount of hydrogen peroxide in composition (b) may typically range from about 0.01 % to about 2%, more typically 0.01 to 1%, but most typically about 0.5 to 1.0%, by weight, based upon the total weight of the composition.

The use of such low levels, even as low as 0.5-1.0 wt.%, of hydrogen peroxide in the 'neutralising conditioner' composition (b), helps to realise the aims of the present invention. The hydrogen peroxide, even at this low concentration, provides good control of malodour arising from the reaction of the cysteamine with the hair.

There are significant advantages in using lower levels of hydrogen peroxide in the neutralising conditioner composition (b). Lower levels of peroxide mean that the straightening treatment using a system of the invention is less damaging to the hair and has less of a detrimental effect upon hair colour.

By 'primary odour neutralising ingredient' it is meant herein that composition (b) does not contain any other, or secondary, odour neutralising ingredient in an amount which is greater than the amount of the hydrogen peroxide which is present in composition (b).

Control of hair frizz, post-treatment, is also important for consumer satisfaction. Components to combat this are therefore included within the present hair styling and straightening system. Many ingredients can be used to condition hair and control frizz in composition (b) accordance with the invention, including, but not limited to, cationic silicone compounds, cationic polymers, cationic surfactants and fatty alcohols.

It has surprisingly been found that a combination of a cationic silicone and a fatty acid ester in the 'neutralising conditioner' composition (b) can produce synergistic effects on frizz control in the hair straightening system of the invention.

Non-limited examples of cationic silicones suitable for use in the hair styling or straightening system include aminopropyl phenyl trimethicone, amodimethicone, bis-hydroxy/methoxy amodimethicone and silicone quaternium-16, or combinations of any two or more thereof.

Typically, the total amount of cationic silicones in composition (b) may typically range from about 0.1% to about 5%, more typically 0.1 to 2.5%, but most typically about 0.1 to 1.0% by weight, based upon the total weight of composition (b).

Non-limited examples of fatty acid esters suitable for use in the straightening system include cocoglycerides, isopropyl myristate, isopropyl palmitate, lauryl palmitate, glyceryl oleate, glyceryl laurate, glyceryl stearate, glyceryl palmitate and glyceryl myristate, or combinations of any two or more thereof.

Typically, the total amount of fatty acid ester in the invention may typically range from about 1% to about 10%, more typically about 1% to about 5%, but most typically about 2 to about 4% by weight, based upon the total weight of composition (b).

Finally, the aims of the present invention can also be realised by straightening the hair after treatment with the system of the invention and drying the hair. Typically, the hair is blow-dried and the straightening is carried out with the aid of straightening irons. The straightening irons act to 'seal-in' the straightening effects of the chemical treatment.

Also provided in accordance with the present invention is a method of treating, styling and straightening hair, comprising applying to the hair a system as described hereinabove, and also further drying and straightening the hair. Typically, the hair is blow-dried and the straightening is carried out with the aid of straightening irons.

Also provided in accordance with the present invention is a use of a system as disclosed hereinabove in a treatment for the straightening of hair. It has been found that the straightening treatment typically works best if it is finished off with a heat treatment with straightening irons, during which the hair exposed to a heat treatment up to about 140-250°C, typically 160-200°C.

Also provided in accordance with the present invention is a kit for a hair styling or straightening treatment, comprising a system as described hereinabove.

Also provided in accordance with the present invention is a method of treating hair, comprising adding to the hair a composition comprising cysteamine in an amount of 4-7 wt%.

Also provided in accordance with the present invention is a use of a composition comprising cysteamine in an amount of 4-7 wt% in a hair straightening treatment.

To date, a composition comprising cysteamine in an amount of 4-7 wt% has not been used in any treatment for the straightening of a person's hair.

According to one embodiment of the invention, the composition may be in the form of a thick cream having a viscosity of between about 15,000 - 25,000 cP (T-bar C, Brookfield Viscometers Ltd) at 10 r.p.m., measured at 20°C.

However, according to another embodiment, the composition may be in the form of a foam, gel, or a mousse.

According to one embodiment of the invention, a typical hair straightening treatment carried out may be carried out as follows. In a first step, the hair is treated with a thick cream formulation (hereinafter referred to as the 'straightening treatment') containing cysteamine as a primary reducing agent. In step two, the hair is treated with a hair conditioner formulation (hereinafter referred to as the 'conditioning neutraliser') containing only low levels of hydrogen peroxide to control malodour, and a blend of aminopropyl phenyl trimethicone and cocoglycerides to control frizz. The hair is then typically blow-dried and then straightened with straightening irons.

The invention will now be described further by way of example with reference to the following examples which are intended to be illustrative only and in no way limiting upon the scope of the invention.

### Examples

### 1. Straightening Formulations

| **Ingredient** | **Inclusion Level (wt.%)** | | | |
|---|---|---|---|---|
| **Example:** | **I** | **II** | **III** | **IV** |
| | | | | |
| Cysteamine | 4.00 | 5.00 | 7.00 | 10.00 |
| Hydroxyethyl Cellulose | 0 | 1.00 | 0.50 | 1.00 |
| Cellulose Gum | 1.00 | 1.00 | 0.50 | 1.50 |
| Cetyl Alcohol | 3.00 | 2.00 | 3.00 | 3.00 |
| Stearyl Alcohol | 3.00 | 4.00 | 3.00 | 3.00 |
| Steareth-20 | 2.00 | 4.00 | 2.00 | 2.00 |
| Glyceryl Stearate | 1.00 | 2.00 | 3.00 | 3.00 |
| Dimethicone | 2.00 | 2.00 | 2.00 | 2.00 |
| Amodimethicone | 0 | 1.00 | 1.00 | 2.00 |
| Polyquaternium-10 | 0.20 | 0.25 | 0.10 | 0.20 |
| Glycerin | 1.00 | 1.00 | 1.00 | 1.00 |
| Hydrolyzed Keratin | 0.20 | 0.20 | 0.10 | 0.10 |
| Disodium EDTA | 0.10 | 0.10 | 0.10 | 0.10 |
| Fragrance | 1.00 | 1.00 | 1.00 | 1.00 |
| Mono-ethanolamine | 1.20 | 1.30 | 1.40 | 1.60 |
| Water | to 100 | to 100 | to 100 | to 100 |

### 2. Neutraliser Formulations

| **Ingredient** | **Inclusion Level (wt.%)** | | | |
|---|---|---|---|---|
| **Example:** | **I** | **II** | **III** | **IV** |
| | | | | |
| Cetrimonium Chloride | 2.00 | 1.00 | 0 | 0 |
| Stearamidopropyl dimethylamine | 0 | 1.00 | 0 | 0 |
| Distearoylethyl Dimonium Chloride | 0 | 0 | 1.0 | 2.0 |
| Hydroxyethyl Cellulose | 1.00 | 1.00 | 0.50 | 1.00 |
| Cellulose Gum | 0 | 1.00 | 0.50 | 1.50 |
| Cetyl Alcohol | 3.00 | 2.00 | 3.00 | 3.00 |
| Stearyl Alcohol | 3.00 | 4.00 | 3.00 | 3.00 |
| Steareth-20 | 2.00 | 4.00 | 2.00 | 2.00 |
| Glyceryl Stearate | 1.00 | 2.00 | 3.00 | 3.00 |
| Dimethicone | 0 | 1.00 | 2.00 | 2.00 |
| Amodimethicone | 0 | 1.00 | 1.00 | 2.00 |
| Aminopropyl Phenyl Trimethicone | 1.00 | 0.50 | 0 | 0 |
| Coconut Oil | 3.00 | 1.00 | 2.00 | 1.00 |
| Rice Bran Oil | 0 | 1.00 | 0.50 | 0.50 |
| Glycerin | 1.00 | 1.00 | 1.00 | 1.00 |
| Hydrolyzed Keratin | 0.10 | 0.20 | 0.10 | 0.10 |
| Tetrasodium Etidronate | 0.30 | 0.10 | 0 | 0.20 |
| Disodium EDTA | 0 | 0.20 | 0.30 | 0.10 |
| Phosphoric Acid | 0 | 1.3 | 0 | 1.3 |
| Citric Acid | 1.2 | 0 | 1.5 | 0 |
| Fragrance | 1.00 | 1.00 | 1.00 | 1.00 |
| Hydrogen Peroxide, 30% | 2.00 | 2.00 | 3.00 | 3.00 |
| Water | to 100 | to 100 | to 100 | to 100 |

### Example 2. Evidence of a straightening effect from the treatment system

Tress tests measured the effects of the effects of a 'straightening treatment', 'neutralising conditioner' and heat treatment with straightening irons. They demonstrate that the treatment system is able to semi-permanently straighten hair.

Experiments used virgin frizzy hair, 20 cm in length (International Hair Importers & Products Inc., USA), cut into 2.0cm wide switches.

Switches were cut in triplicate and washed with 'detox' shampoo (2 ml) for 30 seconds. The switches were then rinsed with warm, running tap water for 1 minute. The switches were then washed and rinsed a second time in the same manner. All switches were then pat dried with paper towelling to remove excess moisture and then gently combed to remove any tangles.

The 'straightening treatment' composition (a) was made according to the following formula, and adjusted to pH 8.5.

| **Material (INCI)** | **% w/w** |
|---|---|
| Water | 75.08 |
| Hydroxyethylcellulose | 0.80 |
| Polyquaternium-10 | 0.25 |
| Glycerin | 1.00 |
| Cetearyl alcohol | 8.00 |
| Cetyl alcohol, glyceryl stearate, ceteth-20, steareth-20 | 3.00 |
| Aminopropyl phenyl trimethicone | 2.00 |
| Parfum | 1.50 |
| Cysteamine Hydrochloride | 5.00 |
| Water (pre-dissolve cysteamine) | 2.00 |
| Monoethanolamine | 1.37 |

The 'neutralising conditioner' composition (b) was made according to the following formula, and adjusted to pH 3.0.

| **Material (INCI)** | **% w/w** |
|---|---|
| Water | 82.00 |
| Hydroxyethylcellulose | 0.70 |
| Stearamidopropyl dimethylamine | 1.00 |
| Citric acid | 0.80 |
| Cetearyl alcohol (50:50) | 2.00 |
| Cetyl alcohol | 3.00 |
| Distearyl dimonium chloride, cetearyl alcohol | 3.00 |
| Quaternium-91 (and) cetrimonium methosulfate (and) cetearyl alcohol | 2.00 |
| Aminopropyl phenyl trimethicone | 2.00 |
| Parfum | 1.50 |
| Hydrogen peroxide | 0.60 |

The 'straightening treatment' was applied to each switch (2 ml) with a syringe and gently rubbed into the switch with the fingers to ensure even distribution. The switches were then combed three times with a wide-toothed comb. After 20 minutes, the switches were rinsed for 1 minute under warm, running tap water and then pat dried with paper towelling.

The 'neutralising conditioner' was applied to each switch (2 ml) with a syringe and again gently rubbed into the switch with the fingers and combed. After 10 minutes, the switches were rinsed for 1 minute under warm, running water and pat dried with paper towelling. Rinsed switches were gently blow-dried (medium force and heat settings) using the fingers to separate out the hairs. Switches were finally straightened with straightening irons.

All switches were left for approximately 24 hours. Each switch was washed with shampoo (CW Frizz Free Shampoo) (2 ml) for 30 seconds and rinsed under warm, running tap water for 1 minute. Shampoo was then applied again to each switch for 30 seconds and rinsed again under warm, running water for 1 minute.

Rinsed switches were pat dried with paper towelling and finally blow-dried gently (medium force and heat settings) using the fingers to separate out the hairs. All switches were then immediately photographed.

For photography and image analysis, switches were hung in front of a light box, which gave a standard white background and minimised shadows. Photography used a digital SLR camera (Canon 450D EOS) with an 18-55 mm lens. Images were analysed using MATLAB R2010a and Image Processing Toolbox (Mathworks Ltd).

Results showed that the switch 'aspect ratio' (length of switch ÷ width at widest section) was 51 % higher (+/- Standard Deviation 11%; n = 3) in treated versus untreated switches. In other words, after treatment and then washing, the hair is clearly semi-permanently straightened.

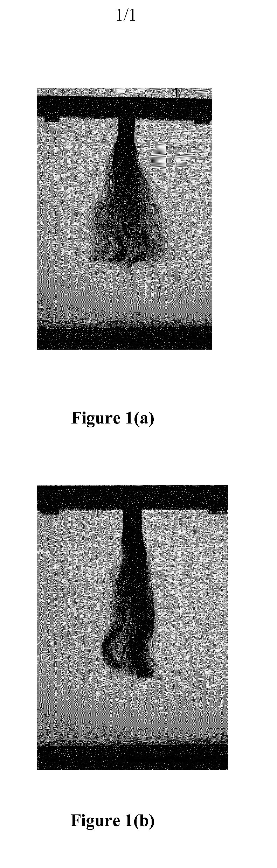

Figures 1(a) and (b) show the effects of the treatment on the hair switches. Figure 1(a) shows a typical control switch that has been shampoo washed and air-dried. Figure 1(b) shows a typical treated switch that has been shampoo washed and air-dried. It is clear that the chemical treatment has made the treated switch noticeably straighter, even after shampooing.

It is of course to be understood that the present invention is not intended to be restricted to the foregoing examples which are described by way of example only.

## Claims

1. A system for styling hair, the system comprising the use of:
a) a composition comprising about 1 to about 10 wt% of cysteamine, or a cosmetically acceptable derivative thereof as a primary reducing agent; and
b) a composition comprising about 0.01% to about 2 wt.% of hydrogen peroxide, a cationic silicone compound, and a fatty acid ester.

2. A system according to claim 1, wherein the cysteamine or a derivative thereof comprises cysteamine in its free form, cysteamine hydrochloride, a monoalkyl aminoethanethiol (e.g. N-acyl cysteamine), a dialkyl aminoethanethiol (e.g. *2-*(dimethylamino) ethanethiol and 2-(diethylamino) ethanethiol) or tert-butyl N-(2-mercaptoethyl) carbamate, or a combination of any two or more thereof;
wherein the monoalkyl aminoethanethiol optionally comprises N-acyl cysteamine, and/or the dialkyl aminoethanethiol comprises 2-(dimethylamino) ethanethiol and/or 2-(diethylamino) ethanethiol.

3. A system according to any preceding claim, wherein the cysteamine or a derivative thereof is present in the composition (a) in an amount of from about 4 to about 7% by weight, based upon the total weight of the composition.

4. A system according to any preceding claim, further comprising in the composition (a) one or more additional, secondary, reducing agents selected from thiols, glyceryl monothioglycolate, cysteine, cysteamine, glutathione, and glyceryl thiolactate, or a combination of any two or more thereof wherein the one or more additional, secondary, reducing agents are optionally present in an amount of from about 10% by weight or less, based upon the total weight of the composition (a).

5. A system according to any preceding claim, wherein the hydrogen peroxide is present in the composition (b) in an amount of about 0.01% to about 2% by weight of the composition, or in an amount of about 0.1 to 1.0% by weight of the composition.

6. A system according to any preceding claim, further comprising one or more additional odour neutralising ingredients in the composition (b), wherein the one or more additional odour neutralising ingredients in the composition (b) are optionally selected from fragrance ingredients, polyhydric phenols, cyclodextrins, cationic polymeric materials, zinc compounds, undecylenic acid, zeolites and silicas, or a combination of any two or more thereof.

7. A system according to any preceding claim, wherein the cationic silicone compound is selected from aminopropyl phenyl trimethicone, modimethicone, bis-hydroxy/methoxy amodimethicone and silicone quaternium-16, or a combination of any two or more thereof, and is optionally present in an amount of about 0.1 to about 5% by weight, based upon the total weight of the composition (b); and wherein the cationic silicone compound optionally comprises aminopropyl phenyl trimethicone.

8. A system according to any preceding claim, wherein the fatty acid ester is selected from cocoglycerides, isopropyl myristate, isopropyl palmitate, lauryl palmitate, glyceryl oleate, glyceryl laurate, glyceryl stearate, glyceryl palmitate and glyceryl myristate, or a combination of any two or more thereof, and is optionally present in an amount of about 1% to about 10% by weight, based upon the total weight of the composition (b).

9. A system according to any preceding claim, further comprising drying and straightening the hair, wherein the straightening optionally involves a heat treatment with straightening irons.

10. A system according to any preceding claim, wherein the system is for straightening a person's hair.

11. A method of styling or straightening a person's hair comprising applying a system according to any preceding claim.

12. Use of a system according to any of claims 1-18 in styling or straightening a person's hair.

13. A kit for a hair styling or straightening treatment, comprising a system according to any of claims 1-18.

14. A method of treating hair, comprising adding to the hair a composition comprising cysteamine in an amount of 4-7 wt%.

15. Use of a composition comprising cysteamine in an amount of 4-7 wt% in a hair straightening treatment.
